# EUROPEAN PATENT APPLICATION

(11) **EP 1 834 580 A1**
(43) Date of publication of application: **19.09.2007**
(21) Application number: 06425182.0
(22) Date of filing: 17.03.2006
(51) Int. Cl.: A61B 5/11

(54) **Apparatus and process for detection and analysis of biomedical data**

(71) Applicant: Universita' degli studi di Bari, 70121 Bari (IT); Politecnico di Bari, 70126 Bari (IT)
(72) Inventor: Quagliarella, Livio c/o Universita' degli studi di Bari, I-70121 Bari (IT); Sasanelli, Nicola c/o POLICLINICO DI BARI, P.zza Giulio Cesare, 11 70124 Bari (IT); Corsi, Francesco c/o Politecnico di Bari, Campus Universitario I-70126 Bari (IT); Cutrone, Natalizia c/o Politecnico di Bari, Campus Universitario I-70126 Bari (IT)
(74) Representative: Trupiano, Federica

(57) **Abstract**

The present invention relates to a portable apparatus and the relative method for the acquisition of data concerning the movement of an individual under examination. The apparatus comprises interface means with a plurality of sensors to be placed on the individual to acquire the data and a memory to store the latter. Advantageously, the apparatus comprises a control unit for processing the acquired data, the control unit assigning each of the interface means to the connection with different sensors that can be interchanged based on algorithms selected by the user.

## Description

The present invention relates to an apparatus for the acquisition and analysis of dynamic biomedical data of individual, particularly an apparatus for the acquisition and analysis of dynamic data concerning the movement of individuals.

By the term "dynamic biomedical data" is meant data to be used for evaluating the functional physical conditions of the individuals under examination, particularly the dynamic data concerning the movement of the individual, both in terms of amplitude and power, which data are then processed for evaluation.

Apparatuses intended to acquire biomedical data from individuals under examination have been available on the market for a long time, particularly data concerning the movement and physical condition of the individuals. These apparatuses are used in the clinical field, such as to evaluate pathologies of the neuromusculoskeletal system, in occupational medicine, post-surgery or post-traumatic rehabilitation, and also in the sports field, in order to evaluate the physical performance of the athletes and the effectiveness of the training programs used.

The processing of the data provides objective indications of the characteristics of the movements analysed and the correspondence of the latter to reference parameters. For example, when the effectiveness of a rehabilitation therapy on a patient is evaluated, the data acquired by the apparatus and processed by an external unit provide a valuable aid to the therapist for an objective evaluation of the rehabilitation level. The acquisition and analysis of the biomedical data may relate to, for example, the evaluation of an athlete's performance in the run, jump, etc., the evaluation of the hand grip force exerted by a patient, the amplitude of rotation of an elbow, knee, etc.

Traditional apparatuses may be either fixed or portable. Fixed apparatuses, which are generally used in hospitals, surgeries and clinics, are bulky and complex to manufacture and develop. Examples of stationary apparatuses are the stereophotogrammetric systems, the electromyographic surface systems and those systems using force platform. The stationary apparatuses strictly require to be used by trained staff, since arranging the sensors is not easy and the data to be acquired are numerous and complex, which cannot be handled by untrained people. Accordingly, the stationary apparatuses are complex, expensive and require highly skilled, dedicated staff.

A further drawback with the stationary apparatuses is that they do not allow acquiring data concerning movements that cannot be carried out by individuals under indoor examination, or however in the environments that are usually destined to the tests with these apparatuses. In this regard, the stationary apparatuses can be hardly applied to the tests to be performed outdoors, such as in sports structures or in the mountains, etc. To overcome this drawback, portable apparatuses have been proposed.

Portable apparatuses acquire data by means of sensors operating on the individual's body. For example, the extent of the movements carried out by the individual is measured by means of kinematic sensors, such as accelerometers, gyroscopes, inertial sensors, etc. The data acquired by the sensors are stored in a suitable memory of the apparatus to be subsequently processed by external units. In other words, the apparatus collects the data from sensors placed on the individual's body. In a later time, the data are transferred to an external processing unit which provides to handling them according to preset algorithms.

An apparatus of this type, which is marketed under the name of ""Physilog", is known for example by Patent Application EP 1195139. This document describes a device suitable to detect parameters connected to postural transitions (with particular reference to "sit-to-stand" or "stand-to-sit") by measuring the transition time, in order to evaluate the risk of fall or monitor the activities carried out in daily life. The analysed signal comes from angular speed sensors (gyroscopes) that may be in combination with accelerometers. The analysis uses the decomposition by means of "wavelet" to calculate the duration of the postural transitions.

This device has thus the disadvantage of being a recorder that can be used only with certain signals, which must however resort to a stationary device to get the data required for the desired evaluation.

Therefore, the need exists for an apparatus that is portable, provides the user with the data required for carrying out the evaluation, i.e. the data obtained by processing the signals from the sensors, and that can be used in a great variety of tests by unskilled people. In other words, the need exists to have an apparatus for the acquisition and processing of dynamic biomedical data in unstructured environments, both in the clinical and sports fields, which is:
- capable of being used by a person with low technical skills,
- cost-effective,
- capable of processing the signals received from the sensors and immediately providing the data required for evaluating the result,
- capable of being adapted to various types of tests also after it has been manufactured.

The object of the present invention is to solve the problems discussed above and provide an apparatus and a method for evaluating dynamic biomedical data which has the characteristics as mentioned above.

This object is achieved by means of the present invention, which provides an apparatus for the acquisition of biomedical data characterized according to claim 1.

As compared with the traditional apparatuses, the apparatus according to the present invention allows a high number of different tests to be carried out on the individual. Traditional portable apparatuses are specially manufactured to be connected to a predefined type of sensors, such as a gyroscopic sensor and an inertial sensor, and subsequent changes are not possible. On the other hand, the apparatus according to the invention can be connected to various types of sensors, which are selected from time to time according to the test to be performed. Upon each use of the apparatus, the interface means are destined by the control unit to be connected to a sensor having known characteristics. In this manner, the apparatus can be interfaced with a plurality of different sensors, even if one or more sensors were not available when the apparatus has been manufactured, or several combinations of the same were not available for testing on the individual.

The instrumental parameters of the individual sensors are stored in the apparatus memory. When a sensor is connected to carry out a test, the control unit recalls the parameters relative to this sensor, which is thereby identified. When the sensor has been identified, the control unit is capable of adjusting, in an autonomous and automatic manner, the gain, offset and duration of the acquisition, the sampling frequency, etc., for the sensor in question.

The apparatus according to the present invention is very versatile as compared with traditional portable apparatuses.

The control unit comprises a mother board that is associated to a processor. A processor suitable for this use is sold by Microchip company, under the code DSPIC30F6012. The algorithms to be selected by the user reside in the processor, as firmware, or they are stored in a suitable memory, either fixed or removable, such as a memory card known under the trade name "Secur Digital", in the form of software programs.

The control unit is configured such that the algorithms can be modified, deleted, or new algorithms can be added to those provided during the manufacturing step.

The algorithms contain a set of instructions and information for the control unit concerning the tests to be carried out by the user.

The interface means comprise one or more input/output cards (I/O) that are provided with communication ports for connection with the sensors. According to the preferred embodiment of the present invention, the apparatus comprises two I/O cards, each being provided with eight (8) channels. The communication ports comprise analogue inputs for movement sensors, inertial sensors (accelerometers and gyroscopes) and pressure sensors, and digital inputs for target sensors, motor revolution or wheel revolution sensors, thermo-couples and other temperature sensors, etc.

The apparatus comprises devices for conditioning the signals acquired through the communication ports. These devices prepare the signals for being handled by the control unit. Preferably, the apparatus comprises a pre-amplifier, one or more filters having the function of restraining the frequency band of the acquired signals to the spectrum used for processing, and an A/D converter.

The sensors can be constrained to the user body to acquire biomedical data concerning movement. Each sensor is characterized by its instrumental parameters, such as sensitiveness, range, gain, offset, etc.

The arrangement of the sensors with the traditional portable apparatuses is preset and cannot be changed by the user, this means that only one type of sensor having known characteristics can be connected to a determined communication port. The apparatus according to the present invention allows a greater flexibility for those connections to be activated with different types of sensors.

The user, who may not be specially trained to use the apparatus, selects the type of test to carry out. Each test is associated with an algorithm. The apparatus informs the user about the type and number of sensors to be used and the connections to carry out, by indicating the exact correspondence between an activated communication port and the corresponding sensor to be connected thereto. In other words, the apparatus identifies the sensor connected to a determined communication port.

Thereby, the user is not required to manually set the parameters relative to the sensors (gain, offset, acquisition parameters, etc.) each time a new test is carried out and the required sensors are connected. The parameters are stored in the apparatus for each type of sensor during the manufacturing step. When the user selects the type of test he/she desires to carry out, the parameters relative to a sensor are recalled by the memory and taken in consideration for the acquisition of the signals.

The apparatus according to the present invention is preferably provided with means for identifying the individual on whom the dynamic tests are carried out. Thereby, the data acquired and processed by the apparatus can be associated with the individual who has been subjected to the tests at any time. For example, the apparatus can be provided with a keyboard. Before the test is carried out, the individual can enter his/her identification data by means of the keyboard.

The apparatus further comprises means for displaying the acquired signals and/or the biomedical data being processed. For example, the apparatus is provided with a screen on which the signals/data are displayed.

The invention further provides a method for the acquisition of biomedical data by means of the above apparatus, characterized according to claim 11.

When the user has selected the test to be carried out, the apparatus performs the following basic functions:
- it indicates the number and type of the sensors to be connected, and optionally to which communication port they require to be connected;
- it sets the acquisition parameters, such as the gain and offset of the sensors, number of tests to be carried out, duration of the acquisition, the sampling frequency, number of channels involved in the test, etc.;
- requires the identification of the individual who is subjected to the test;
- provides to the acquisition of the signals from the sensors;
- stores the acquired signals;
- displays on the screen one or more signals in real time during acquisition;
- processes the signals to obtain data of biomedical interest, such as functional indexes;
- provides the user with the results obtained from the processing.

Each step requires the control unit to be activated for managing the apparatus peripherals. These peripherals may comprise a keyboard, a screen, a digital control potentiometer for manually adjusting the gain and offset of the sensors, a mass memory, a printer, a USB interface, etc.

The signals acquired by the sensors are handled by said conditioning means before being processed by the control unit. The signals are subjected to filtering, digitalization, interpolation, and when required they are mathematically handled with operators of convolution, self-correlation, Fourier transform, etc.

The apparatus according to the present invention has a number of advantages as compared with the traditional portable apparatuses that are used to acquire dynamic biomedical data from individuals under examination. First, the apparatus according to the invention is not limited to the acquisition of data, but allows a quick processing of the latter.

Furthermore, according to the invention is very versatile, since it allows the acquisition and processing of the signals supplied by different types of sensors arranged from time to time based on the different tests to be carried out. The communication ports of the equipment, to which the sensors are connected, are adapted to recognize and be connected to a particular sensor among the different sensors that can be used. In other words, the apparatus according to the present invention can be connected to different types of sensors without requiring the hardware to be accordingly modified, but simply by arranging the communication ports in a suitable manner.

The arrangement of the sensors, i.e. the connection of the sensors to the apparatus interface means is very simple. The same apparatus, in fact, provides to guide the user in the selection of the sensors to be activated for a determined test and in the arrangement of the relative connections.

In practice, the user activates the apparatus and thus carries out the selection of the algorithm relative to the test he/she desires to carry out. The apparatus, based on this algorithm, changes the destination of the input/output channels and advises the user about which channel a determined sensor requires to be connected to. Thereby, the apparatus can also be used by a user who is not specially trained, with clear advantages in terms of distribution of the apparatus.

The apparatus does not require to be connected to external processing means, such as PCs, consoles, or the like, since it provides to process the signals that are collected by the sensors being connected thereto in an autonomous manner. Thereby, the user has an immediate feedback about the test performed.

The apparatus can be re-programmed after it has been manufactured. The algorithms stored in the control unit can be modified, deleted, rewritten to take into account any change in the tests to be carried out. More algorithms can be added to take into account more tests the user desires to carry out.

The apparatus according to the invention can be manufactured at low cost. Furthermore, it can be manufactured with lightweight and small-sized components, with clear advantages as regards the wearability of the apparatus. The apparatus ensures a great freedom of movement to the user, not only in the simulated testing conditions, but also in the actual testing conditions.

Further aspects and the advantages of the present invention will be better understood from the description below, which is to be considered by way of a non-limiting example with reference to the annexed figures, in which:
- Fig. 1 is a general diagram of the apparatus according to the present invention;
- Fig. 2 is a general diagram of a first component of the apparatus according to the present invention;
- Fig. 3 is a general diagram of a second component of the apparatus according to the present invention;
- Fig. 4 is a general diagram of a third component of the apparatus according to the present invention;
- Fig. 5 is an exploded view of an apparatus according to the present invention.
- Fig. 6 is a block diagram of the method according to the present invention.

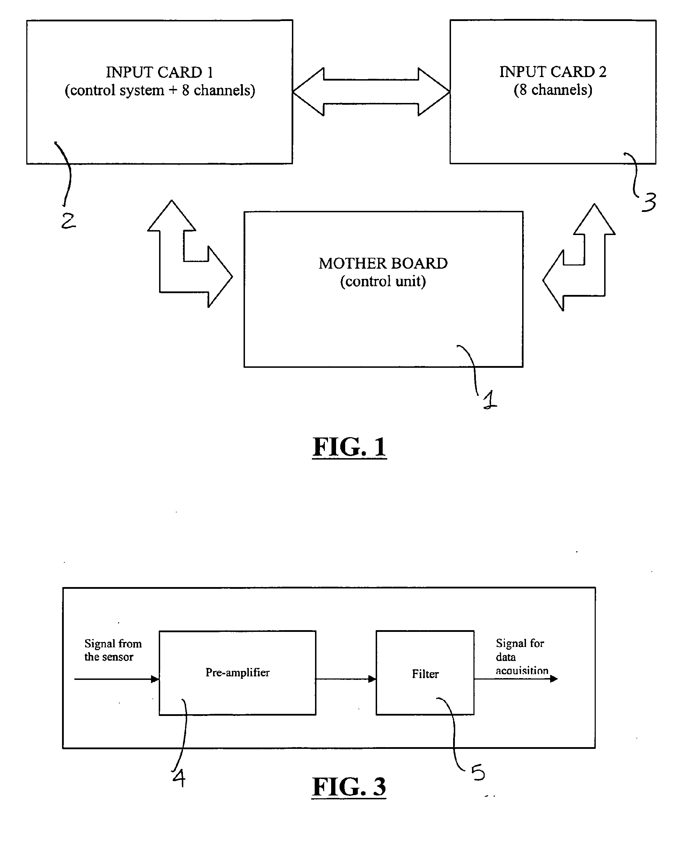

With reference to Fig. 1, there is illustrated a general diagram of the structure of an apparatus according to the present invention. The apparatus comprises a control unit 1 and interface means 2 and 3 with a plurality of external sensors (not shown).

The structure as illustrated provides the apparatus with a greater versatility than the traditional portable apparatuses used for the acquisition of biomedical data from individuals. The control unit 1 interacts with the interface means 2 and 3 such that their operation is adapted according to the desired test to be performed. The arrows indicate that the interaction is provided between the control unit and the interface means, and vice versa, but also between the interface means themselves.

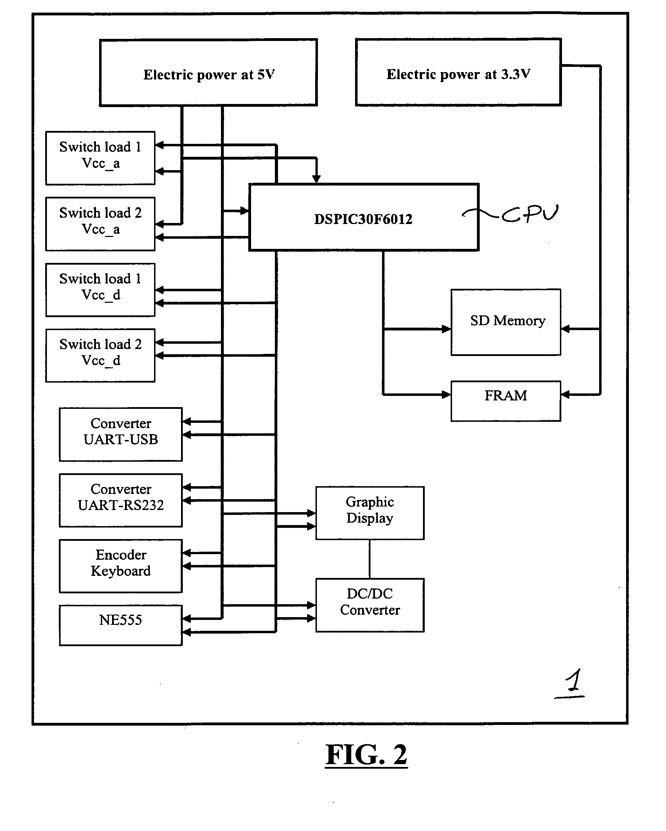

In the preferred embodiment, the apparatus comprises two input cards 2 and 3 (Fig. 1) and a control and storage unit (Fig. 1 and 2), i.e. the mother board 1, that are connected to each other. Each of the two input cards 2 and 3 is provided with eight channels for connection with the sensors, which can be configured either in a single-ended or differential mode, such as to have sixteen channels available, which are completely independent.

This configuration is to be preferred to the solution in which one only card with sixteen channels is used, since it allows reducing the size of the printed circuit and thus the bulk of the device, in favour of the wearability of the apparatus.

Fig. 2 shows a diagram of the structure of the mother board 1 to be used with this invention. As may be seen, on the mother board there are provided both the control unit CPU (such as the processor sold by Microchip company under the code DSPIC30F6012) to which the input cards 1 and 2, and other peripherals are connected. In the embodiment illustrated, the unit CPU is supplied by a suitable line with a voltage of 3,3V, whereas the cards 2 and 3 and the other peripherals are supplied with a voltage of 5V by means of a second line.

The components supplied with 5V are:
- Switch load;
- UART-USB converter;
- UART-RS232 converter;
- Keyboard encoder;
- Graphic display;
- Dc/Dc converter;
- NE555.

The components supplied with 3.3V are:
- SD mass memory;
- FRAM.

Analogue inputs of the input cards 2 and 3 supplied with a voltage ranging between 0 and 5 V are used for the movement sensors, inertial sensors (accelerometers and gyroscopes) and pressure sensors. Digital inputs of the input cards 2 and 3 are used for the target, motor revolution and wheel revolution sensors and thermocouples or RPT and temperature sensors. Preferably, the input cards 2 and 3 are provided with differential inputs (with the possibility of reading both positive and negative voltage values) and/or inputs with adjustable power supply (such as for extensometric sensors).

The control unit CPU communicates with all the components of the mother board, and furthermore, via bus (12C and SPI) communicates with the input cards and three I/O Expanders and the digital potentiometers, respectively. Voltage dividers have been introduced, which are capable of converting the signal from one voltage level to another, whenever this is required in order to obtain the communication between components with different power supply, such as between the memory SD and control unit.

A FRAM memory (Ferroelectric Random Access Memory) is further shown in Fig. 2 , which functions as a buffer and communicates with the control unit CPU and memory SD by means of the same BUS SPI that allows the communication between the control unit CPU and memory SD. The FRAM memory operates as a "buffer memory" and stores the data at a speed consistent with the control unit CPU. To this operation there follows the transfer of the same data to the memory SD at a speed consistent therewith.

The unit CPU has the task of processing the signals supplied by the sensors connected to the input cards 2 and 3. The result of the processing is a plurality of biomedical data concerning the individual being subjected to the test, particularly data concerning the movement of the individual or portions of the same.

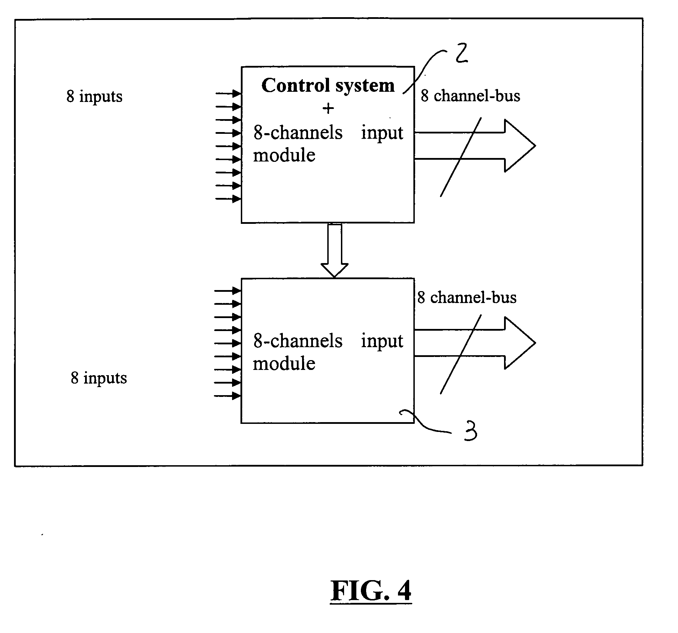

The two input cards 2 and 3 are outlined in Fig. 4. As may be seen, the first input card 2 comprises both an input module with 8 channels (similarly to the second input card 3) and a control system. The input modules converge on an analogue bus with 8 channels. The 8-channel bus is connected to 8 analogue inputs of the control unit CPU.

Each module connects to the bus only when the control unit CPU must detect the signal from the sensor connected to at least one of the channels of the module, in practice to communication ports of the module. After this detection has been carried out, the bus is cleared.

Each module consists of 8 identical channels, plus a section in common to all the channels, which consists of three I/O Expander elements. The I/O Expander elements are controlled by the unit CPU and have the task of selecting one of the sixteen channels that are available on the two input cards 2 and 3. In the embodiment illustrated, the I/O Expander elements are inserted in the first input card 2 but also control the other eight channels of the second input card.

The input cards 2 and 3 also deal with the signal conditioning. Fig. 3 shows a block diagram of the handling of the signals from the sensors. The preamplifier 4 has the function of amplifying the level of the signal before the "noise" is added thereto, which is caused by the other components of the apparatus. Preferably, the preamplifier 4 is of a linear type to prevent that distortions may be introduced on the signal, and low-noise. Furthermore, the pre-amplifier 4 adapts the level of the signal received from the sensor to the best operating conditions of the subsequent stages, particularly of the analogue-to-digital converter. The latter converts the type of signal. For example, if the signal provided by the sensor is a current signal and the components required to handle this signal operate on voltage signals, the converter carries out the current-to-voltage conversion.

The filter 5 restrains the frequency band of the incoming signal to the usable spectrum, thereby eliminating the disturbances. The filter further carries out the anti-aliasing of the signal. By the term "anti-aliasing" (or pre-sampling) is meant the elimination from the sampled signal of the different components (alias) not contained in the original signal, thereby limiting the spectrum to the usable band.

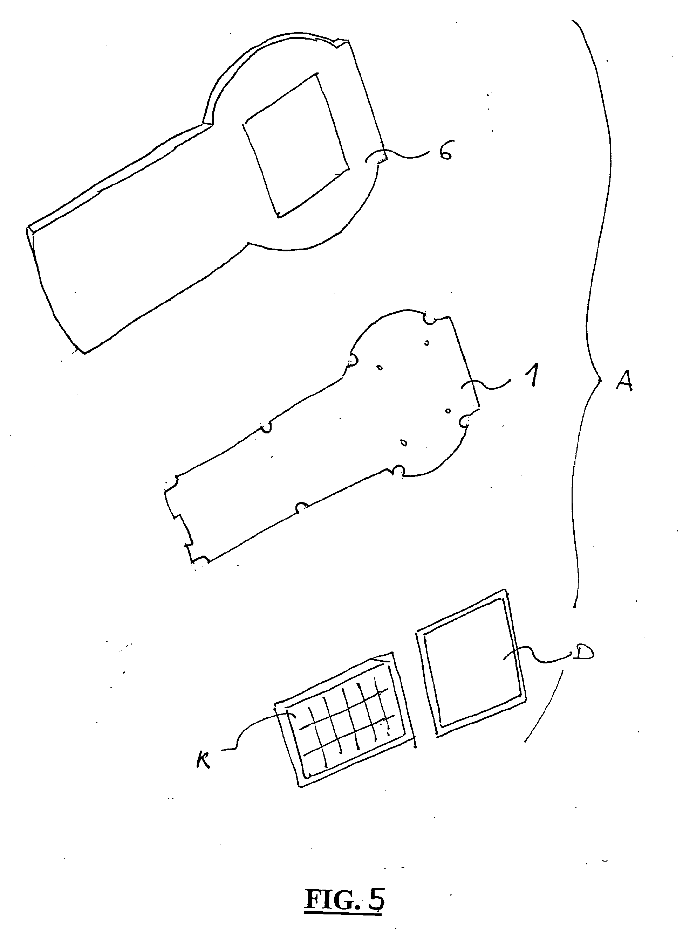

Fig. 5 is an exploded view of an apparatus A according to the present invention. The mother board 1 with the control unit CPU can be inserted in a portable case, i.e. having such a size as to be "worn", or such as to be pocket-sized, etc. The apparatus is completed by a keyboard K and a screen D as a user interface.

A plurality of algorithms resides, in the form of software or firmware, in the control unit CPU and/or in the memory SD. The algorithms provide a plurality of information and instructions to the control unit for managing the input ports 2 and 3 and for managing and processing the acquired signals. The apparatus A allows a number of different dynamic tests to be carried out on the individual. An algorithm is available for each test.

The instrumental parameters of the individual sensors are stored either in the memory SD of the apparatus or in the control unit CPU. When a sensor is connected to the apparatus A to carry out a test, the control unit CPU identifies the sensor by means of these parameters. When the sensor has been identified, the control unit CPU is capable of adjusting, in an autonomous and automatic manner, the gain, offset and duration of the acquisition, the sampling frequency, etc., for the sensor in question.

The operation of the apparatus A according to the present invention can be explained with reference to the following example. It is assumed that the apparatus is arranged to carry out four dynamic tests on the subject. For each test, the apparatus is provided with a corresponding algorithm containing the instructions and information required by the control unit to carry out the test. The user can select among the four following tests:
1. test p1;
2. test p2;
3. test p3;
4. test p4.

Each test p1-p4 requires using at least one sensor. The selection of the test p3 by the user activates the following functions/steps (macro-functions):
1. Setting of the acquisition parameters, such as:
   a) Gain and offset;
   b) Number of tests to be carried out;
   c) Acquisition time;
   d) Sampling frequency;
   e) Number of channels involved in the test;
2. Identification of the individual;
3. Acquisition of the signal;
4. Storage of the acquired signal;
5. Optional display of the signal in real-time.
6. Processing of the signal to determine the biomedical parameters of interest;
7. Calculation of the functional indexes;
8. Providing the user with the processed biomedical data, such as by means of peripherals such as a printer, a screen , etc., or by transferring the data to an external PC.

Each step or function can require the implementation of secondary steps or the use of peripherals interfaced to the control unit CPU such as, for example, a keyboard, a display, a digital-control potentiometer, a mass memory, a printer, a USB interface, etc.

The conditioning of the signals from the sensors can provide the steps of: filtering, digitalization, interpolation, handling with mathematical operators of convolution, self-correlation, Fourier transform (FFT or inverse FFT).

The steps (micro-functions) subsequent to the selection of the test by the user are:
- the control unit CPU selects the number and type of sensors based on the algorithm corresponding to the selected test and informs the user about the instrumental parameters that are recalled for the selected sensors;
- the control unit carries out the calibration of the sensors in an automatic manner by adjusting the gain and offset;
- input of the identification data by the user, such as by means of the keyboard K;
- acquisition of the signals generated by the sensors;
- storage of the signals acquired in the memory SD or CPU of the apparatus A;
- optionally display in real-time of one or more acquired signals (use of the display);
- processing of the signal and calculation of the biomedical data of interest, functional indexes, etc;
- providing the user with the processing results, such as on a paper medium by means of printing or a video, by means of the screen D.
- optional stocking of the acquired signals and biomedical data processed with external units, such as a PC (download of the data by means of USB interfaces, Bluetooth, etc.);

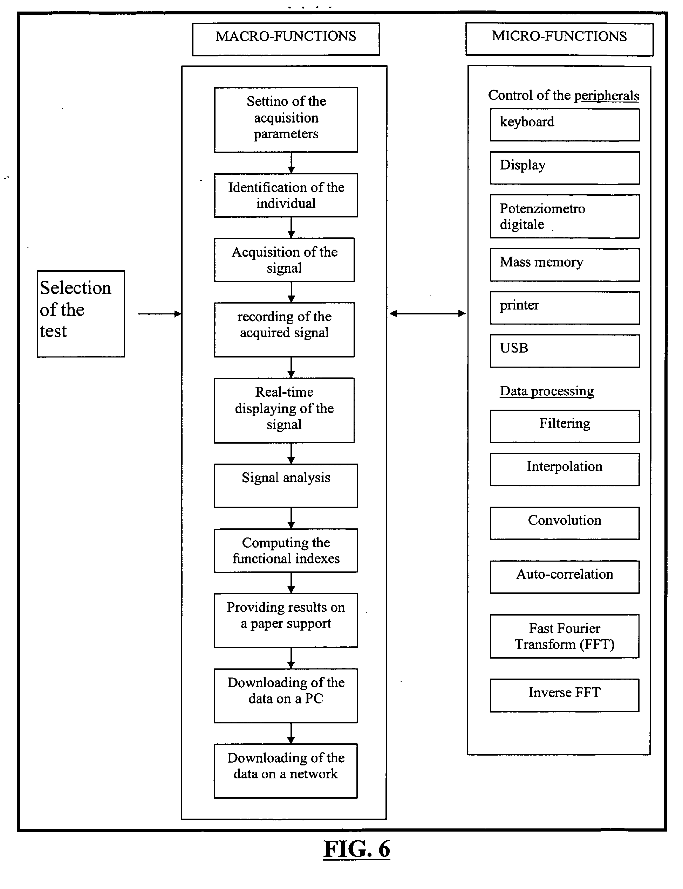

The block diagram shown in Fig. 6 summarizes the steps and functions concerning the operation of the apparatus A according to the present invention.

Advantageously, the apparatus A has an internal structure that can be defined as "modular", since the unit CPU controls the operation of the various components, such as the input cards 2 and 3, peripherals K and D, memory SD, etc. Thereby, the operation of the apparatus A can be adapted to new testing, also in a later time after manufacturing.

It is sufficient that new algorithms are inserted or those residing in the apparatus A are changed to take the new testing into account. The new algorithms will contain the instrumental parameters to be used for identifying new sensors and their instructions of use.

In contrast with the traditional portable apparatuses, the portable apparatus A is not limited to the acquisition of data, but it also allows a quick processing of the same. The apparatus A does not require to be connected to external processing means, such as PCs, consoles, or the like, since it provides to process the signals that are collected by the sensors being connected thereto in an autonomous manner. Thereby, the user has an immediate feedback about the test performed.

Furthermore, the apparatus A allows the acquisition and processing of the signals supplied by different types of sensors being arranged from time to time based on the various tests to be carried out. The apparatus identifies the connected sensors in an autonomous manner, and automatically calibrates the same. Furthermore, the apparatus A provides to guide the user in the selection of the sensors to be activated for a determined test and in the arrangement of the relative connections.

The apparatus can be re-programmed after it has been manufactured. The algorithms stored in the control unit can be modified, deleted, rewritten to take into account any change that may occur in the tests to be carried out. More algorithms can be added to take into account more tests that the user desires to carry out.

The apparatus according to the invention can be manufactured at low cost. Furthermore, it can be manufactured with lightweight and small-sized components, with clear advantages in terms of wearability of the apparatus.

## Claims

1. A portable apparatus for acquiring dynamic biomedical data on an individual, comprising means for connection to a plurality of sensors and means for acquiring and storing the signals generated by said sensors, **characterized in that** it further comprises a control unit containing:
- means for identifying the sensors to be connected to said apparatus depending on the test to be carried out;
- means for controlling the operation of said sensors after they have been connected to said apparatus based on instrumental parameters of said sensors, said parameters being stored in said apparatus;
- means for processing the signals generated from said sensors and generating a datum indicative of the signal received;
- means for storing said data of the processed signals and/or means for displaying said data;
said control unit assigning said interface means to the connection with different sensors that can be interchanged depending on the type of data to be acquired.

2. The apparatus according to claim 1, **characterized in that** said interface means comprise a plurality of communication ports to be programmed by said control unit for connection to sensors of a different type.

3. The apparatus according to claim 1 or 2, **characterized in that** said control unit comprises a mother board and a processor, and said interface means comprise one or more I/O cards being provided with communication ports.

4. The apparatus according to claim 3, **characterized in that** said interface means comprise two input cards, each with eight channels, that are connected to said mother board.

5. The apparatus according to any preceding claim, **characterized in that** it comprises an analog-to-digital converter for the digitalization of the signals acquired from said sensors.

6. The apparatus according to any preceding claim, **characterized in that** said means for processing the signals generated by said sensors and generating an indicative datum of the received signal process said signals according to the instructions that are implemented in a set of algorithms stored in said means.

7. The apparatus according to any preceding claim, **characterized in that** said algorithms contain instructions for said control unit concerning a plurality of tests that can be carried out.

8. The apparatus according to any preceding claim, **characterized in that** it further comprises means for identifying an individual whose data have to be acquired.

9. A method for acquiring and processing data concerning the movement of an individual under examination by means of the apparatus according to the preceding claims, **characterized in that** it comprises the steps of:
- entering in said apparatus the data acquisition request by means of a dynamic test;
- comparing the request being made with the instructions stored in said control unit;
- selecting the instructions corresponding to the test to be carried out;
- connecting the sensors provided for said test to interface means according to the correspondence provided by said control unit;
- setting the gain and offset of the sensors connected to the apparatus;
- acquiring the signals from the connected sensors;
- processing said signals to extrapolate said data;
- providing the result of said processing.

10. The method according to claim 9, **characterized in that** it further comprises the step of processing said signals for calculating one or more functional indexes concerning the user.

11. The method according to claim 9 or 10, **characterized in that** it further comprises at least one of the following steps:
- storing said signals and/or said data on a removable memory;
- identifying the user;
- displaying at least one of said acquired signals;
- displaying at least one of said data concerning the user's movement.

12. Use of the apparatus according to any preceding claim for acquiring and processing dynamic biomedical data.
